# EUROPEAN PATENT APPLICATION

(11) **EP 4 843 507 A1**
(43) Date of publication of application: **25.11.2026**
(21) Application number: 26179658.5
(22) Date of filing: 19.05.2026
(51) Int. Cl.: A61B 8/06, A61B 8/00

(54) **ULTRASOUND DIAGNOSTIC APPARATUS & STORAGE MEDIUM**

(30) Priority: 20.05.2025 JP 2025083870
(71) Applicant: Canon Kabushiki Kaisha, Tokyo 146-8501 (JP)
(72) Inventor: IKEDA, Hayato, Tokyo, 146-8501 (JP)
(74) Representative: Marks & Clerk LLP

(57) **Abstract**

An ultrasound diagnostic apparatus (10) according to an embodiment includes an ultrasound probe (5), an acquisition unit (111), an execution unit (115), a detection unit (112), a first display control unit (116), and a second display control unit (117). The ultrasound probe (5) transmits ultrasound to a subject and receives ultrasound reflected by the subject. The acquisition unit (111) successively acquires, on the basis of reception signals of the ultrasound received by the ultrasound probe (5), first ultrasound data in a time direction, the first ultrasound data representing a fluid moving in the body of the subject. The execution unit (115) executes, on the basis of the first ultrasound data successively acquired by the acquisition unit (111), processing of generating second ultrasound data having higher spatial resolution for the fluid than the first ultrasound data. The detection unit (112) detects a trigger for executing the processing. The first display control unit (116) displays, on a display, first ultrasound image based on at least the sets of first ultrasound data, during a period from detection of the trigger until a condition determined beforehand is satisfied. The second display control unit (117) displays, on the display, second ultrasound image based on at least the second ultrasound data, after the condition determined beforehand is satisfied.

## Description

### FIELD

Embodiments disclosed in the present specification are related to ultrasound diagnostic apparatuses, medical information processing apparatuses, and storage media.

### BACKGROUND

Techniques for improving image quality of ultrasound images including fine flow channels and fluids (for example, blood flow and contrast agents) have been known conventionally. For example, Patent Literature 1 discloses generating images representing a boundary of a flow channel (for example, a shape of a blood vessel) by removing clutter included in consecutive ultrasound reception signals in a predetermined time period and averaging fluid signals after this removal of the clutter over a predetermined time period. Furthermore, Patent Literature 2 discloses performing super-resolution processing that improves resolution (pixel density) of ultrasound images and sharpens peaks of speckle patterns in the ultrasound images, and displaying a fluid flow channel with high image quality by superimposing a plurality of peak-sharpened speckle patterns within a predetermined time period.

In these techniques, an image higher in image quality is able to generated as the number of frames used increases, but in processing at an initial stage after image collection is started, image quality is deteriorated because the number of frames is still small.

### SUMMARY OF INVENTION

An ultrasound diagnostic apparatus provided in an aspect of the present embodiment includes an ultrasound probe that transmits ultrasound to a subject and receives ultrasound reflected by the subject, an acquisition unit that successively acquires, on the basis of reception signals of the ultrasound received by the ultrasound probe, first ultrasound data in a time direction, the first ultrasound data representing a fluid moving in the body of the subject, an execution unit that executes processing of generating, on the basis of the first ultrasound data successively acquired by the acquisition unit, second ultrasound data having higher spatial resolution for the fluid than the first ultrasound data, a detection unit that detects a trigger for executing the processing, a first display control unit that displays, on a display, first ultrasound image based on at least the first ultrasound data, during a period from detection of the trigger until a condition determined beforehand is satisfied, and a second display control unit that displays, on the display, second ultrasound image based on at least the sets of second ultrasound data, after the condition determined beforehand is satisfied.

The first display control unit may display, on the display, as the first ultrasound image, combined image obtained by combining the first ultrasound data and the second ultrasound data, the combined image having smaller proportions of the second ultrasound data than the second ultrasound data used in generation of the second ultrasound image.

The second display control unit may display, on the display, as the second ultrasound image, combined image obtained by combining the first ultrasound data and the second ultrasound data, the combined image having smaller proportions of the first ultrasound data than the first ultrasound data used in generation of the first ultrasound image.

The first display control unit may successively display, on the display, the first ultrasound images obtained by combining the first ultrasound data and the second ultrasound data at ratios corresponding to elapse of time, during a period from the detection of the trigger until a predetermined time period elapses.

The first display control unit may successively display, on the display, the first ultrasound images obtained by combining the first ultrasound data and the second ultrasound data at ratios corresponding to the numbers of frames, during a period from the detection of the trigger until the number of frames represented by the first ultrasound data reaches a predetermined number.

The first display control unit may successively display, on the display, the first ultrasound images obtained by combining the first ultrasound data and the second ultrasound data at ratios each corresponding to an image quality index value, during a period from the detection of the trigger until the image quality index value represented by the second ultrasound data enters a predetermined state.

The detection unit may detect, as the trigger, operation for selecting a mode in which the second ultrasound image are displayed, and the first display control unit may display the first ultrasound image on the display during a period from the operation for selecting the mode until the condition is satisfied.

The detection unit may detect, as the trigger, detection of a stationary state of the ultrasound probe, and the first display control unit may display the first ultrasound image on the display during a period from the detection of the stationary state of the ultrasound probe until the condition is satisfied.

The detection unit may detect, as the trigger, a stopped state of body motion represented by ultrasound data of the subject acquired through preprocessing of the first ultrasound data, and the first display control unit may display the first ultrasound image on the display during a period from detection of the stopped state of the body motion until the condition is satisfied.

The ultrasound diagnostic apparatus may include a generation unit that generates filter information based on identification information identifying positions of the fluid in the first ultrasound data, wherein the execution unit may generate the second ultrasound data using the filter information that has been subjected to weighting according to times elapsed since start of generation of the second ultrasound data or the numbers of frames of data used in generation of the second ultrasound data.

The generation unit may generate the filter information that has been weighted such that the shorter the time elapsed or the smaller the number of frames of data, the smaller the value generated.

The acquisition unit may acquire, as the first ultrasound data, added data each obtained by addition processing of a plurality of frames of data that are obtained by transmission and reception of the ultrasound by the ultrasound probe and that are consecutive in the time direction.

A medical information processing apparatus provided in an aspect of the present embodiment includes an acquisition unit that successively acquires, in a time direction, first ultrasound data representing a fluid moving in the body of a subject, an execution unit that executes processing of generating, on the basis of the first ultrasound data successively acquired by the acquisition unit, second ultrasound data having higher spatial resolution for the fluid than the first ultrasound data, a detection unit that detects a trigger for executing the processing, a first display control unit that displays, on a display, first ultrasound image based on at least the first ultrasound data, during a period from detection of the trigger until a condition determined beforehand is satisfied, and a second display control unit that displays, on the display, second ultrasound image based on at least the second ultrasound data, after the condition determined beforehand is satisfied.

A storage medium provided in an aspect of the present embodiment that non-transitorily stores a program for causing a computer to execute successively acquiring, in a time direction, first ultrasound data representing a fluid moving in the body of a subject, executing processing of acquiring, on the basis of the first ultrasound data successively acquired, second ultrasound data having higher spatial resolution for the fluid than the first ultrasound data, detecting a trigger for executing the processing, displaying, on a display, first ultrasound image based on at least the first ultrasound data, during a period from detection of the trigger until a condition determined beforehand is satisfied, and displaying, on the display, second ultrasound image based on at least the second ultrasound data, after the condition determined beforehand is satisfied.

### BRIEF DESCRIPTION OF THE DRAWINGS

FIG. 1 is a block diagram illustrating an example of a configuration of an ultrasound diagnostic apparatus according to a first embodiment;
FIG. 2 is a flowchart illustrating a processing procedure executed by the ultrasound diagnostic apparatus according to the first embodiment;
FIG. 3 is a flowchart illustrating a processing procedure for a first ultrasound data generation mode according to the first embodiment;
FIG. 4 is a diagram for illustration of an example of data processing according to the first embodiment;
FIG. 5 is a flowchart illustrating a processing procedure for a second ultrasound data generation mode according to the first embodiment;
FIG. 6 is a diagram for illustration of an example of data processing according to the first embodiment;
FIG. 7 is a diagram illustrating an example of extraction processing according to the first embodiment;
FIG. 8 is a flowchart illustrating a processing procedure executed by the ultrasound diagnostic apparatus according to the first embodiment;
FIG. 9 is a diagram for illustration of an example of data processing according to the first embodiment;
FIG. 10A is a diagram illustrating an example of first ultrasound data according to the first embodiment;
FIG. 10B is a diagram illustrating an example of filter information according to the first embodiment;
FIG. 11 is a diagram illustrating an example of second ultrasound data according to the first embodiment;
FIG. 12 is a diagram for illustration of an example of combining processing according to the first embodiment; and
FIG. 13 is a diagram for illustration of an example of data processing according to a second embodiment.

### DETAILED DESCRIPTION

Embodiments of an ultrasound diagnostic apparatus, a medical information processing apparatus, and a program, according to the present application will hereinafter be described in detail by reference to the appended drawings. The ultrasound diagnostic apparatus, the medical information processing apparatus, and the program, according to the present application are not to be limited by the embodiments described hereinafter.

### First Embodiment

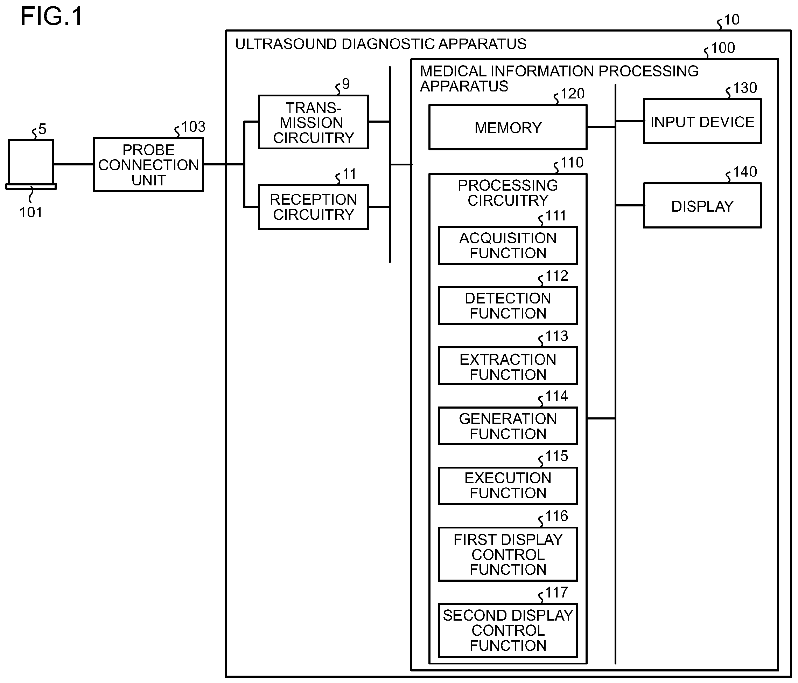

FIG. 1 is a block diagram illustrating an example of a configuration of an ultrasound diagnostic apparatus 10 according to a first embodiment. The ultrasound diagnostic apparatus 10 is an apparatus that generates ultrasound data on the basis of reception signals (reflected wave signals) received from an ultrasound probe 5. The ultrasound diagnostic apparatus 10 illustrated in FIG. 1 is an apparatus that is capable of generating two-dimensional ultrasound data on the basis of reception signals received from a one-dimensional ultrasound probe 5 (an ultrasound probe having transducers arranged one-dimensionally) and is capable of generating three-dimensional ultrasound data on the basis of reception signals received from a two-dimensional ultrasound probe 5 (an ultrasound probe having transducers arranged two-dimensionally).

The ultrasound probe 5 transmits ultrasound to a subject and receives ultrasound reflected by the subject. The ultrasound probe 5 is, for example, an electronic scanning probe, and has a plurality of transducers 101 arranged one-dimensionally or two-dimensionally at a distal end thereof. The transducers 101 are piezoelectric elements (electromechanical conversion elements) that perform mutual conversion between electric signals (voltage pulse signals) and ultrasound waves (acoustic waves). The ultrasound probe 5 transmits ultrasound from the plurality of transducers 101 to a subject and receives reflected ultrasound from the subject by means of the plurality of transducers 101. The reflected acoustic waves indicate differences in acoustic impedance within the subject. In a case where ultrasound pulses transmitted are reflected by moving blood flow or a surface of a heart wall, for example, reflected ultrasound experiences a frequency shift due to the Doppler effect, the frequency shift being dependent on a velocity signal component of the moving object in relation to the ultrasound transmission direction.

A probe connection unit 103 connects the ultrasound probe 5 and transmits and receives ultrasound to and from the ultrasound probe 5. The probe connection unit 103 may connect the ultrasound probe 5 by a wired or wireless means. In a case where a wired means is used, the probe connection unit 103 has a connector unit (receptacle) to connect a connector (plug) of the ultrasound probe 5. In a case where a wireless means is used, the probe connection unit 103 has a communication unit to perform wireless communication with the ultrasound probe 5.

The ultrasound diagnostic apparatus 10 includes transmission circuitry 9, reception circuitry 11, and a medical information processing apparatus 100.

The transmission circuitry 9 is a transmission unit that outputs pulse signals (drive signals) to the plurality of transducers 101. Applying the pulse signals to the plurality of transducers 101 with time differences causes the plurality of transducers 101 to transmit ultrasound with different delay times, and a transmitted ultrasound beam is thereby formed. Selectively changing the transducers 101 (that is, the transducers 101 to be driven) to which the pulse signals are applied and changing the delay times (application timings) of the pulse signals enable control of the direction and focus of the transmitted ultrasound beam. Successively changing the direction and focus of the transmitted ultrasound beam causes an observed region inside the subject to be scanned. Furthermore, a transmitted ultrasound beam having plane waves (with a far focus) or diffuse waves (with a focus point in a direction opposite to the ultrasound transmission direction with respect to the plurality of transducers 101) may be formed by changing the delay times of the pulse signals. Or one transducer or some of the plurality of transducers 101 may be used to form a transmitted ultrasound beam. By transmitting pulse signals having a predetermined drive waveform to the transducers 101, the transmission circuitry 9 causes transmitted ultrasound to be generated in the transducers 101, the transmitted ultrasound having a predetermined transmission waveform.

The reception circuitry 11 is a reception unit that inputs, as reception signals, electric signals output from the transducers 101 that have received reflected ultrasound. The reception signals are input to processing circuitry 110. With respect to this embodiment, analog signals output from the transducers 101 and digital data sampled (digitally converted) from the analog signals will be referred to as reception signals with no particular distinction.

The medical information processing apparatus 100 is connected to the transmission circuitry 9 and the reception circuitry 11 and executes processing of signals received from the reception circuitry 11 and control of the transmission circuitry 9. The medical information processing apparatus 100 includes the processing circuitry 110, a memory 120, an input device 130, and a display 140.

The memory 120 includes, for example, a semiconductor memory element, such as a random access memory (RAM) or a flash memory, a hard disk, and/or an optical disk. The memory 120 is a memory to store data, such as image data for display, the image data having been generated by the processing circuitry 110. Furthermore, the memory 120 is also capable of storing reception signals (reflected wave signals) output by the reception circuitry 11. In addition, as required, the memory 120 stores: control programs for performing ultrasound transmission and reception, image processing, and display processing; and various data, such as diagnostic information (for example, patient IDs and physicians' findings), diagnostic protocols, and various body marks.

The input device 130 receives various instructions and input of information from an operator. The input device 130 includes, for example, an input interface device, such as a trackball, a switch button, a mouse, a keyboard, a touch pad through which input operation is performed by a touch on an operation surface thereof, a touch monitor having a display screen and a touch pad integrated, a non-contact input circuit using an optical sensor, or a voice input circuit. The input interface device is connected to the processing circuitry 110 described later, converts input operation received from an operator into an electric signal, and outputs the electric signal to the processing circuitry 110. According to the present specification, the input interface device is not necessarily an input interface device having a physical operating part, such as a mouse or a keyboard. For example, examples of the input interface device also include processing circuitry that receives an electric signal corresponding to input operation from an external input device provided separately from the apparatus and outputs this electric signal to the processing circuitry 110.

Under control of the processing circuitry 110, the display 140 displays a graphical user interface (GUI) for receiving input of imaging conditions, and various images. The display 140 includes, for example, a display interface device, such as a liquid crystal display.

By controlling each unit of the ultrasound diagnostic apparatus 10, the processing circuitry 110 controls the entire ultrasound diagnostic apparatus 10. For example, the processing circuitry 110 functions as an acquisition function 111, a detection function 112, an extraction function 113, a generation function 114, an execution function 115, a first display control function 116, and a second display control function 117, by executing programs stores in the memory 120. The processing circuitry 110 is implemented by, for example, a processor. The acquisition function 111 is an example of an acquisition unit. The detection function 112 is an example of a detection unit. The generation function 114 is an example of a generation unit. The execution function 115 is an example of an execution unit. The first display control function 116 is an example of a first display control unit. The second display control function 117 is an example of a second display control unit. The processing circuitry 110 is described as being implemented singly in FIG. 1, but a plurality of independent processors may be combined for implementation by the multiple processors. Furthermore, the processing circuitry 110 may include an independent circuit dedicated to a specific function, such as an application specific integrated circuit (ASIC).

Furthermore, the term, "processor", used above means, for example: a central processing unit (CPU); a graphical processing unit (GPU); or circuitry, such as an application specific integrated circuit (ASIC) or a programmable logic device (for example, a simple programmable logic device (SPLD), a complex programmable logic device (CPLD), or a field programmable gate array (FPGA)). The processor implements functions by reading and executing the programs stored in the memory 120.

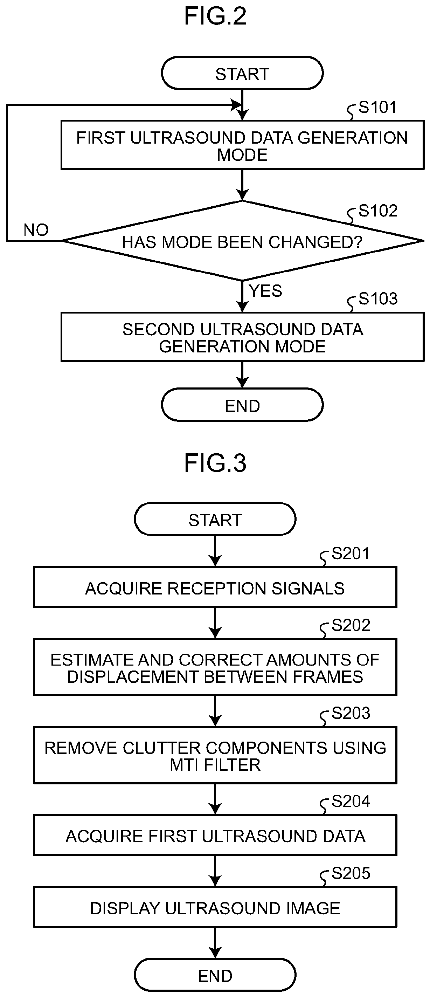

The ultrasound diagnostic apparatus 10 configured as described above stably provides highly visible images. Details of operation of the ultrasound diagnostic apparatus 10 will be described hereinafter. FIG. 2 is a flowchart illustrating a processing procedure executed by the ultrasound diagnostic apparatus 10 according to the first embodiment. FIG. 2 illustrates an overview of the processing procedure at the ultrasound diagnostic apparatus 10.

As illustrated in FIG. 2, the processing circuitry 110 in the ultrasound diagnostic apparatus 10 according to the first embodiment executes processing in a first ultrasound data generation mode and causes the display 140 to display ultrasound images based on first ultrasound data (Step S101). The first ultrasound data generation mode is mode in which the first ultrasound data representing a fluid (for example, blood flow) moving in the body of a subject are generated. The processing circuitry 110 causes the display 140 to display, on the basis of the first ultrasound data mentioned above, the ultrasound images representing the fluid moving in the body of the subject.

While the ultrasound images are being displayed in the first ultrasound data generation mode, the processing circuitry 110 determines whether or not the mode has been changed (Step S102). Specifically, the processing circuitry 110 determines whether or not the mode has been changed to a second ultrasound data generation mode that is a mode in which second ultrasound data having higher spatial resolution for the fluid than the first ultrasound data are generated. For example, an operator performs operation for changing the mode from the first ultrasound data generation mode to the second ultrasound data generation mode via the input device 130.

In a case where the mode has been changed (Step S102, Yes), the processing circuitry 110 transitions to the second ultrasound data generation mode and starts processing of generating the second ultrasound data (Step S103). In a case where the mode has not been changed (Step S102, No), the processing circuitry 110 continues operating in the first ultrasound data generation mode until display of ultrasound images is stopped.

Details of the first ultrasound data generation mode and the second ultrasound data generation mode will be described next in order. FIG. 3 is a flowchart illustrating a processing procedure for the first ultrasound data generation mode according to the first embodiment. As illustrated in FIG. 3, the processing circuitry 110 controls the transmission circuitry 9 and the reception circuitry 11 by means of a control function not illustrated in the figure, to cause an ultrasound scan to be executed by means of the ultrasound probe 5 and to acquire reception signals (for example, CH data) (Step S201). Specifically, the processing circuitry 110 starts control to collect, at a predetermined frame rate, a plurality of frames of data consecutive in a time direction (a plurality of frames of data within a predetermined time period) acquired by execution of an ultrasound scan. A frame of data includes amplitude values (signal values) of signals corresponding to respective pixels constituting one image (frame).

The processing circuitry 110 performs phasing addition and quadrature detection processing on the collected reception signals by means of a signal processing function not illustrated in the figure. Phasing addition processing is processing of adding up reception signals from the plurality of transducers 101 by varying delay times and weights for the respective transducers 101, and is also called delay and sum (DAS) beam forming. The quadrature detection processing is processing of converting the reception signals into in-phase signals and quadrature signals in a baseband frequency band and thereby acquiring measurement data representing IQ data or absolute values of the IQ data, for example. In addition, the reception signals may be subjected to, for example, adaptive beam foaming, model-based processing, or processing using machine learning.

When the plurality of frames of data have been collected, the processing circuitry 110 estimates amounts of displacement of tissue caused by body motion of the subject between the frames of data, and corrects the frames of data on the basis of results estimated (Step S202). Specifically, the processing circuitry 110 calculates amounts of displacement of tissue caused by, for example, body motion between the frames, from the plurality of frames of data, and corrects the frames of data on the basis of the amounts of displacement calculated.

The processing circuitry 110 generates blood flow data (power Doppler data) obtained by extracting information derived from blood flow in the measurement data mentioned above. For example, the processing circuitry 110 applies a moving target indicator (MTI) filter to each of the frames of data collected (Step S203). Information (clutter) derived from tissue remaining stationary or tissue small in motion between the frames is thereby reduced and the information derived from the blood flow (blood flow signal component) is thereby extracted. A filter having fixed filter information, such as a Butterworth infinite impulse response (IIR) filter or a polynomial regression filter, may be used as the MTI filter. The MTI filter may be an adaptive filter that uses eigenvalue decomposition or singular value decomposition, to change coefficients in accordance with an input signal.

The processing circuitry 110 may decompose frames of data into a plurality of bases using eigenvalue decomposition or singular value decomposition, remove information derived from tissue by taking out specific bases, and extract information derived from blood flow. Furthermore, the processing circuitry 110 may find a velocity vector for each coordinate in reception signal data by using a method, such as the vector Doppler method, the speckle tracking method, or the Vector Flow Mapping method, to find a blood flow vector representing magnitude and a direction of blood flow. Any method that enables extraction of information derived from blood flow included in frames of data (measurement data) or removal of information derived from tissue may be adopted, other than the methods mentioned above as examples. The processing circuitry 110 may also generate B-mode data by performing envelope detection processing or logarithmic compression processing, on measurement data, the B-mode data representing signal intensity at each point in an observation region as brightness.

The acquisition function 111 successively acquires, in the time direction, first ultrasound data representing a fluid moving in the body of a subject, on the basis of reception signals of ultrasound received by the ultrasound probe 5. Specifically, the acquisition function 111 acquires ultrasound data based on the frames of data after Step S203. For example, the acquisition function 111 acquires the plurality of frames of data after Step S203, performs addition processing between the frames, and thereby acquires first ultrasound data (Step S204).

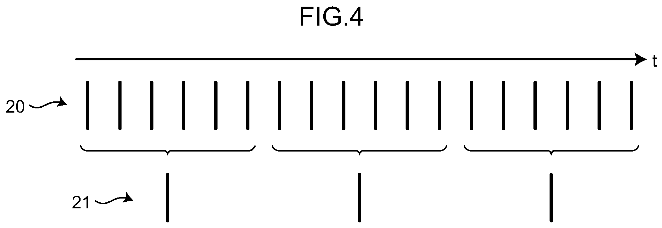

For example, as illustrated in FIG. 4, firstly, the processing circuitry 110 reduces information derived from tissue (tissue signal component (clutter)) from a plurality of frames of data consecutive in the time direction, and thereby respectively acquires frames of data 20 (for example, power Doppler data) that have been obtained by extracting information derived from a fluid (for example, a blood flow signal component) and that have not been subjected to addition processing yet.

The acquisition function 111 acquires sets of first ultrasound data 21 by performing addition processing on the plurality of frames of data 20 consecutive in the time direction for each predetermined number of frames (FIG. 4 illustrates a case where the predetermined number is six). The predetermined number of frames (the number of packets) referred to herein corresponds to, for example, several frames to about 100 frames. In the first ultrasound data generation mode, the processing circuitry 110 successively acquires the sets of first ultrasound data 21 according to collection of the frames of data, and successively displays ultrasound images based on the sets of first ultrasound data 21 (Step S205).

The sets of first ultrasound data 21 each have a higher signal-noise ratio (SN ratio) than the respective frames of data 20 before the addition. The sets of first ultrasound data 21 (added data) are, for example, sets of blood flow data (power Doppler data) in which information derived from blood flow is emphasized. Furthermore, in the sets of first ultrasound data 21, fluid signals after clutter removal have been averaged over a predetermined time period, and the sets of first ultrasound data 21 thereby represent a boundary of a flow path (for example, a shape of a blood vessel). However, the sets of first ultrasound data 21 include: signal values (amplitude values) representing a speckle pattern generated by constructive interference and destructive interference between ultrasound waves reflected from the fluid; and signal values representing other noise (for example, artifacts) generated instantaneously or intermittently on a time axis.

As described by reference to FIG. 2, after a mode change is detected, the ultrasound diagnostic apparatus 10 transitions to the second ultrasound data generation mode. For example, the detection function 112 detects, as a trigger, operation for selecting a mode for displaying second ultrasound images (high-definition ultrasound images) based on sets of second ultrasound data (data obtained by high-definition processing) having higher spatial resolution for the fluid moving in the subject than the sets of first ultrasound data. In this case, for example, the detection function 112 determines whether or not a button for transitioning to a mode in which high-definition processing is performed has been pressed by an operator.

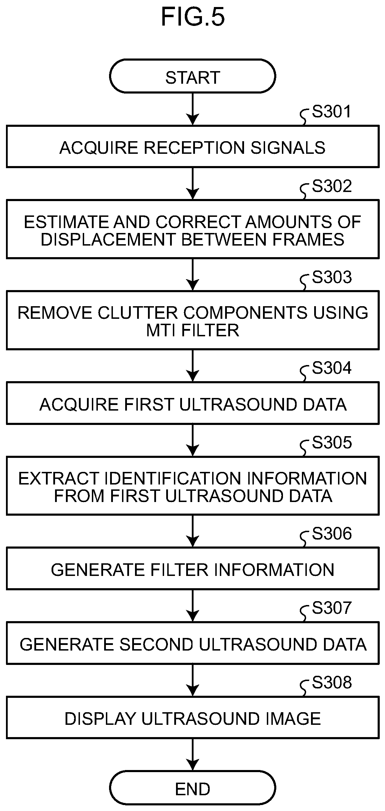

In the second ultrasound data generation mode, the processing circuitry 110 generates the sets of second ultrasound data having higher spatial resolution for the fluid (for example, blood flow) than the sets of first ultrasound data, by performing processing using, as analysis frames, sets of first ultrasound data 21 acquired in the same manner as in the first ultrasound data generation mode. FIG. 5 is a flowchart illustrating a processing procedure for the second ultrasound data generation mode, according to the first embodiment. Steps S301 to S304 in FIG. 5 correspond to processing similar to that of Steps S201 to S204 in FIG. 3, and detailed description thereof will thus be omitted.

When the detection function 112 detects a mode change, as illustrated in FIG. 5, the processing circuitry 110 acquires reception signals (for example, CH data) (Step S301), estimates amounts of displacement between frames, and performs correction (Step S302). Furthermore, the processing circuitry 110 applies an MTI filter to each of frames of data collected (Step S303), performs addition processing between the frames, and thereby acquires sets of first ultrasound data (Step S304).

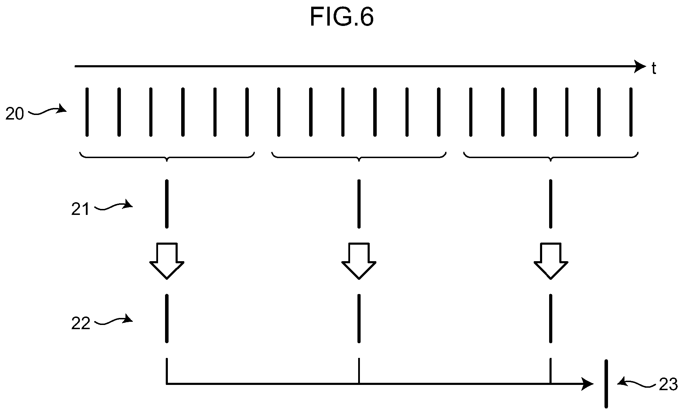

For example, as illustrated in FIG. 6, the processing circuitry 110 reduces information derived from tissue (a tissue signal component (clutter)) from a plurality of frames of data consecutive in the time direction, and thereby respectively acquires frames of data 20 (power Doppler data) that have been obtained by extracting information derived from a fluid (for example, a blood flow signal component) and that have not been subjected to addition processing yet. The acquisition function 111 acquires sets of first ultrasound data 21 by performing addition processing on the plurality of frames of data 20 consecutive in the time direction for each predetermined number of frames (FIG. 6 illustrates a case where the predetermined number is six).

Subsequently, in the second ultrasound data generation mode, the extraction function 113 respectively extracts sets of identification information 22 from the sets of first ultrasound data 21 successively generated, as illustrated in FIG. 6, by extracting signal components representing the fluid moving in the body of the subject from the sets of first ultrasound data (Step S305). The sets of identification information are each a set of information identifying, for each position in a frame (image) represented by the first ultrasound data 21, a magnitude relation between a signal value corresponding to the position and signal values corresponding to surroundings of the position.

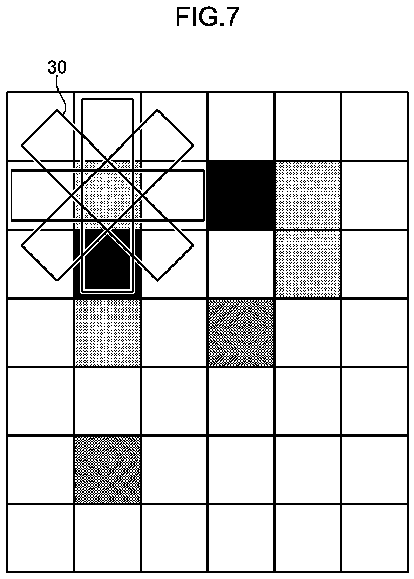

For example, as illustrated in FIG. 7, the extraction function 113 places a kernel 30 at a position of interest in a frame (image) represented by a set of first ultrasound data 21, and compares magnitude relations between a signal value corresponding to the position of interest and signal values corresponding to image positions within a range over which the kernel 30 has been placed (surroundings of the position of interest). For example, for each position in a frame, the extraction function 113 acquires, as identification information, information identifying whether or not that position has a signal value larger than signal values corresponding to surroundings of that position. That is, the identification information may also be referred to as identification information identifying a position having a relatively large signal value (local peak position) in a local range of the frame.

Specifically, as illustrated in FIG. 7, in a case where a kernel based on a vertical-to-horizontal ratio of 3:1 is used, the extraction function 113 places the center (a second cell) of the kernel 30 at a position of interest and identifies whether or not a signal value corresponding to the position is larger than signal values corresponding to positions at both ends (a first cell and a third cell) of the kernel 30. For example, the extraction function 113 represents the signal value corresponding to the position of interest as "1" in a case where the signal value is larger than the signal values corresponding to the surroundings of the position, and represents the signal value corresponding to the position as "0" in a case where the signal value is smaller than the signal values corresponding to the surroundings of the position. The extraction function 113 extracts a set of identification information for the entire frame (image) represented by a set of first ultrasound data 21 by extracting identification information for each position in the frame represented by the set of first ultrasound data 21. That is, through this extraction processing, the set of first ultrasound data 21 is converted to a binary image in which each pixel of the frame (image) is represented by "0" or "1", and amplitude values (signal values) of signals corresponding to the respective pixels constituting the set of first ultrasound data 21 are thereby lost.

FIG. 7 illustrates an example in which a set of identification information is extracted by placing the kernel 30 in four directions, a vertical direction, a horizontal direction, a diagonally upper right direction, and a diagonally upper left direction, for each position in the frame represented by the set of first ultrasound data, but the shape, size, and placement of the kernel are not limited to this example. Furthermore, in the example illustrated in FIG. 7, targets are extracted in pixel units, but a set of identification information may be extracted for respective positions in a frame in group units each having a plurality of pixels. In a case where a set of identification information is extracted in group units each having a plurality of pixels, an average value or an integrated value of signal values at respective positions constituting each group may be used as a signal value corresponding to each position in that frame.

In the second ultrasound data generation mode, the generation function 114 further generates filter information corresponding to the respective positions in the frames, on the basis of the set of identification information 22 extracted from each of the sets of first ultrasound data 21 (Step S306). The filter information is information statistically representing local peak positions of the fluid in the individual frames over a time period corresponding to the plurality of frames. The local peak positions of the fluid are each a position having a relatively high signal value in a local range of the frame. For example, as illustrated in FIG. 6, the processing circuitry 110 generates filter information 23 using sets of identification information 22 corresponding to three frames. The filter information 23 is determined according to values represented by the sets of identification information 22 at positions (coordinate (x, y)) corresponding to one another among the frames. For example, in a case where the values are all "1" for the three frames at mutually corresponding positions in the sets of identification information 22, the filter information for that position is determined as "1". In a case where "1" is acquired once and "0" is acquired twice for the three frames at mutually corresponding positions, the filter information for that position is determined as "0.33". In a case where "0" is acquired for all of the three frames at mutually corresponding positions, the filter information for that position is determined as "0".

As described above, the filter information 23 is set to a larger value as the number of times that "1" is acquired for mutually corresponding positions among a plurality of sets of identification information 22 within a predetermined time period increases. That is, as the number of times that "1" is acquired for mutually corresponding positions in respective sets of identification information 22 increases, signal values each larger than those of the surroundings are consecutively obtained at that position, indicating a higher likelihood that a fluid is present. Therefore, a weighting coefficient serving as the filter information is set to a high value ("1") for such a position. In contrast, in a case where "0" is included at mutually corresponding positions of the respective sets of identification information 22, there is a high likelihood that noise (such as artifacts) generated instantaneously or intermittently along the time axis is present at that position. Therefore, for such a position, as the number of "0"s increases, a weighting coefficient serving as the filter information is set to a lower value (e.g., "0.67," "0.33," or "0").

In the second ultrasound data generation mode, the execution function 115 generates a set of second ultrasound data on the basis of the filter information 23 and at least one of the sets of first ultrasound data 21 (Step S307). For example, the execution function 115 generates a set of second ultrasound data by multiplying, signal values (amplitude values) representing respective positions in a frame represented by the most recent set of first ultrasound data 21, by filter information (weighting coefficients) set for the respective corresponding positions in the filter information 23. That is, the set of second ultrasound data is a set of data obtained by transforming the set of first ultrasound data 21 using the filter information 23. The processing circuitry 110 then causes the display 140 to display an ultrasound image based on the set of second ultrasound data (Step S308). In the second ultrasound data generation mode, as compared with the first ultrasound data generation mode, the above described processing using filter information enables an ultrasound image to be displayed, the ultrasound image in which clutter has been further reduced and separability of adjacent blood flows has been improved.

In the above described second ultrasound data generation mode, a high-resolution ultrasound image depicting a fluid, such as blood flow, is able to be displayed, but at an initial stage after the second ultrasound data generation mode is started, because sufficient frames of data to be used for analysis have not been collected yet, an effect of sufficiently achieving high resolution is unable to be obtained. The ultrasound diagnostic apparatus 10 according to the embodiment thus performs processing of improving (correcting) image quality of second ultrasound data after transitioning to the second ultrasound data generation mode and until a condition determined beforehand is satisfied, thereby enabling stable provision of highly visible images.

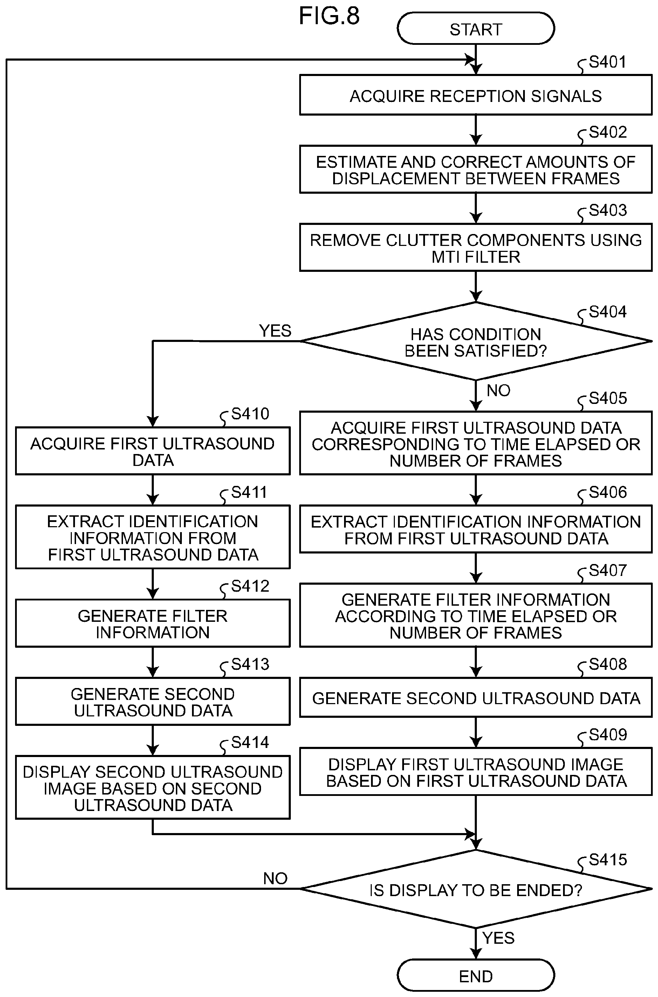

FIG. 8 is a flowchart illustrating a processing procedure executed by the ultrasound diagnostic apparatus 10 according to the first embodiment. Steps S401 to S403 in FIG. 8 correspond to processing similar to that of Steps S201 to S203 in FIG. 3, and detailed description thereof will thus be omitted. Furthermore, Steps S410 to S413 in FIG. 8 correspond to processing similar to that of Steps S304 to S307 in FIG. 5, and detailed description thereof will thus be omitted.

When the detection function 112 detects a mode change, as illustrated in FIG. 8, the processing circuitry 110 acquires reception signals (for example, CH data) (Step S401), estimates and corrects amounts of displacement between frames (Step S402), and applies an MTI filter to each frame of data collected (Step S403).

In the second ultrasound data generation mode according to the embodiment, after the processing of Step S403, the acquisition function 111 determines whether or not a condition has been satisfied (Step S404). Specifically, after transitioning to the second ultrasound data generation mode, the acquisition function 111 determines whether or not a condition enabling obtainment of an effect of sufficiently achieving high resolution has been satisfied. This condition determined beforehand includes, for example, elapse of a predetermined time period or acquisition of a predetermined number of frames. For example, the acquisition function 111 determines whether or not the number of frames (18 frames in FIG. 6) for generating filter information described by reference to FIG. 6 has been acquired after a mode transition, or a time period required for collection of that number of frames has elapsed.

In a case where the condition has not been satisfied (Step S404, No), the acquisition function 111 adjusts the number of frames of data to be used for a set of first ultrasound data according to a time elapsed since the detection of the mode change or the number frames of data collected, and acquires sets of first ultrasound data (S405). That is, the acquisition function 111 acquires sets of first ultrasound data by changing the number of frames to be added according to the time elapsed since the detection of the mode change or the number of frames of data collected.

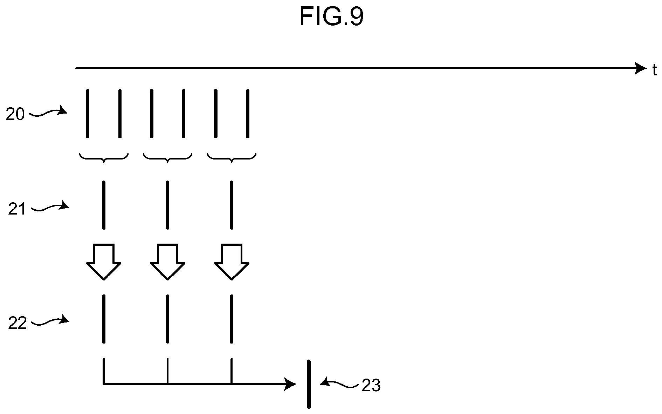

For example, as illustrated in FIG. 9, in a case where a plurality of frames of data 20 from which clutter components have been removed by the processing of Step S403 and which are consecutive in the time direction have been collected for six frames, the acquisition function 111 acquires sets of first ultrasound data 21 obtained by performing addition in units of two frames. The acquisition function 111 increases the number of frames to be added as the number of frames of data 20 collected increases (for example, in a case where nine frames have been collected, the acquisition function 111 may perform addition in units of three frames, and in a case where twelve frames have been collected, the acquisition function 111 may perform addition in units of four frames).

That is, the acquisition function 111 acquires the time elapsed or the number of frames of data collected since the detection of the mode change, and generates sets of first ultrasound data 21 while dynamically changing, on the basis of a result acquired, the number of frames to be used.

The extraction function 113 extracts signal components representing the fluid moving in the body of the subject from the sets of first ultrasound data. Specifically, as illustrated in FIG. 9, the extraction function 113 extracts sets of identification information 22 from the sets of first ultrasound data 21 successively generated (Step S406).

The extraction function 113 extracts the sets of identification information according to the numbers of frames that were added when the sets of first ultrasound data 21 were acquired. Specifically, the smaller the number of frames added, the smaller the numerical value determined by the extraction function 113, the numerical value representing a local peak position. For example, as described by reference to FIG. 6, in a case where the identification information of a local peak position is represented by "1" for sets of first ultrasound data obtained by addition performed in units of six frames, identification information representing a local peak position with a numerical value smaller than "1" is extracted for five frames or less.

For example, as illustrated in FIG. 9, in a case where sets of first ultrasound data 21 are acquired by performing addition in units of two frames, if a signal value corresponding to a position of interest is larger than signal values corresponding to surroundings of that position, the position of interest is represented by, for example, "0.2" in the above described processing using the kernel 30. In contrast, in a case where the signal value corresponding to a position of interest is smaller than the signal values corresponding to surroundings of that position, the position of interest is represented by "0", for example. Furthermore, in a case where sets of first ultrasound data 21 are acquired by performing addition in units of three frames, if the signal value of a position of interest is larger than the surrounding signal values, the position of interest is represented by "0.4", and if the signal value is smaller, the position of interest is represented by "0". Furthermore, in a case where sets of first ultrasound data 21 are acquired by performing addition in units of four frames, if the signal value of a position of interest is larger than the surrounding signal values, the position of interest is represented by "0.6" and if the signal value is smaller, the position of interest is represented by "0". Furthermore, in a case where sets of first ultrasound data 21 are acquired by performing addition in units of five frames, if the signal value of a position of interest is larger than the surrounding signal values, the position of interest is represented by "0.8" and if the signal value is smaller, the position of interest is represented by "0".

The above described example is just an example, and the values for the case where the signal value corresponding to a position of interest is larger than signal values corresponding to the surroundings of the position may be set at any values. Furthermore, different values may be set per any number of frames, instead of being set per frame. Furthermore, the initial processing in the case where sets of first ultrasound data are generated in units of six frames has been described above with respect to the example, but the number of frames set is not necessarily six and any number of frames may be set.

Furthermore, the case where sets of identification information corresponding to the numbers of frames added are extracted has been described above with respect to the example, but sets of identification information corresponding to times elapsed since detection of a mode change may be extracted. In that case, sets of identification information are extracted such that the shorter the time elapsed, the smaller the numerical value of the local peak position, and the longer the time elapsed, the larger the numerical value of the local peak position.

The generation function 114 generates filter information based on sets of identification information identifying the positions of a fluid in sets of first ultrasound data. Specifically, on the basis of sets of identification information 22 extracted from sets of first ultrasound data 21, the generation function 114 generates filter information corresponding to each position in the frame, for each set of first ultrasound data. The generation function 114 generates the filter information according to the time elapsed or number of frames (Step S407). Specifically, the generation function 114 generates the filter information weighted such that the shorter the time elapsed or the smaller the number of frames of data, the smaller the value generated.

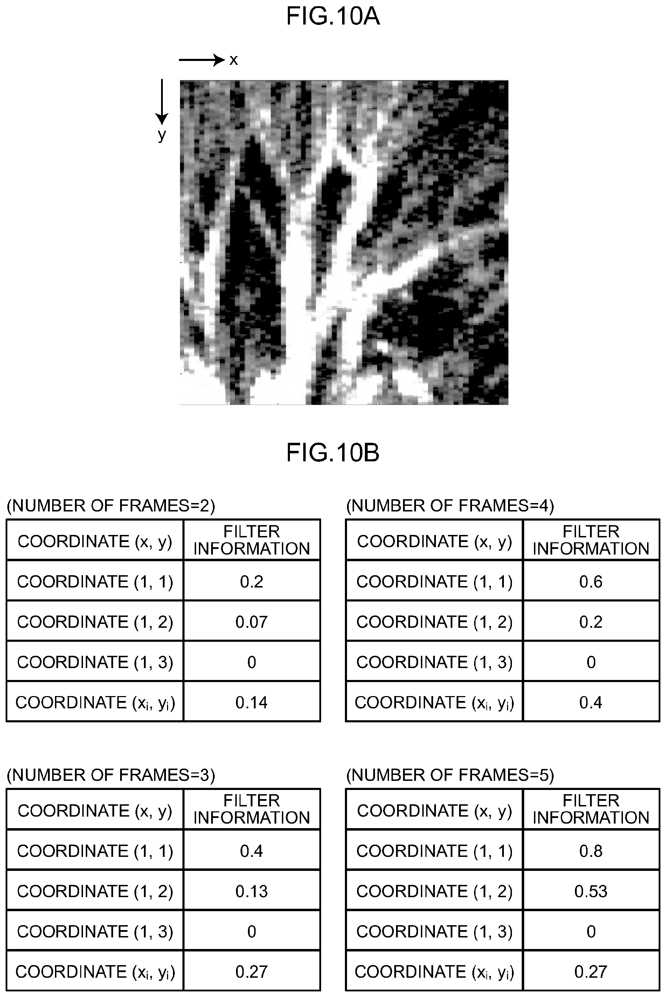

For example, the generation function 114 generates filter information on the basis of identification information extracted at each position (coordinate (x, y)) in a frame (a set of first ultrasound data) illustrated in FIG. 10A. The identification information extracted by the extraction function 113 is identification corresponding to the number of frames added, and the filter information generated by the generation function 114 is thus weighted such that the smaller the number of frames added, the smaller the value generated.

For example, as illustrated in FIG. 10B, in a case where the number of frames added is "2" (the case illustrated in FIG. 9), the filter information at a position where "0.2" is obtained for all of the three frames for the mutually corresponding positions in the sets of identification information 22 is "0.2". Furthermore, in a case where "0.2" is obtained once among the three frames for the mutually corresponding positions, the filter information for that position is "0.07"; in a case where "0.2" is obtained twice, the filter information for that position is "0.14"; and in the case where "0" is obtained for all of the three frames, the filter information for that position is "0".

Similarly, in a case where the number of frames is "3" for which the numerical value of the local peak position is represented by "0.4", filter information having each coordinate represented by "0", "0.13", "0.27", or "0.4" is generated according to the number of times "0.4" is obtained. Furthermore, in a case where the number of frames is "4" for which the numerical value of the local peak position is represented by "0.6", filter information having each coordinate represented by "0", "0.2", "0.4", or "0.6" is generated according to the number of times "0.6" is obtained. Furthermore, in a case where the number of frames is "5" for which the numerical value of the local peak position is represented by "0.8", filter information having each coordinate represented by "0", "0.27", "0.53", or "0.8" is generated according to the number of times "0.8" is obtained. At the initial stage after the processing is started, the generation function 114 thus generates filter information that approaches "1" as the number of frames added increases.

On the basis of sets of first ultrasound data successively acquired by the acquisition function 111, the execution function 115 executes processing of generating second ultrasound data having higher spatial resolution for a fluid than the first ultrasound data. Specifically, the execution function 115 generates the second ultrasound data based on the filter information (Step S408). Specifically, the execution function 115 generates the second ultrasound data using the filter information that has been subjected to weighting according to the times elapsed since the start of the generation of the second ultrasound data or the numbers of frames of data used in the generation of the second ultrasound data. For example, the execution function 115 generates a set of second ultrasound data by multiplying, signal values (amplitude values) of respective positions in a frame represented by a set of first ultrasound data 21, by filter information (weighting coefficients) set for the respective corresponding positions in filter information. That is, the execution function 115 generates a set of second ultrasound data by multiplying the signal values in a set of first ultrasound data for which filter information has been generated, by the filter information. The execution function 115 thereby generates the sets of second ultrasound data whose image quality has been corrected using the filter information subjected to weighting according to the times elapsed or the numbers of frames of data.

For example, the execution function 115 multiplies signal values of respective positions in a set of first ultrasound data for which the number of frames added is "2", by the filter information illustrated in FIG. 10B (the number of frames = 2). Furthermore, for example, the execution function 115 multiplies signal values of respective positions in a set of first ultrasound data for which the number of frames added is "3", by the filter information illustrated in FIG. 10B (the number of frames = 3). Furthermore, for example, the execution function 115 multiplies signal values of respective positions in a set of first ultrasound data for which the number of frames added is "4", by the filter information illustrated in FIG. 10B (the number of frames = 4). Furthermore, for example, the execution function 115 multiplies signal values of respective positions in a set of first ultrasound data for which the number of frames added is "5", by the filter information illustrated in FIG. 10B (the number of frames = 5).

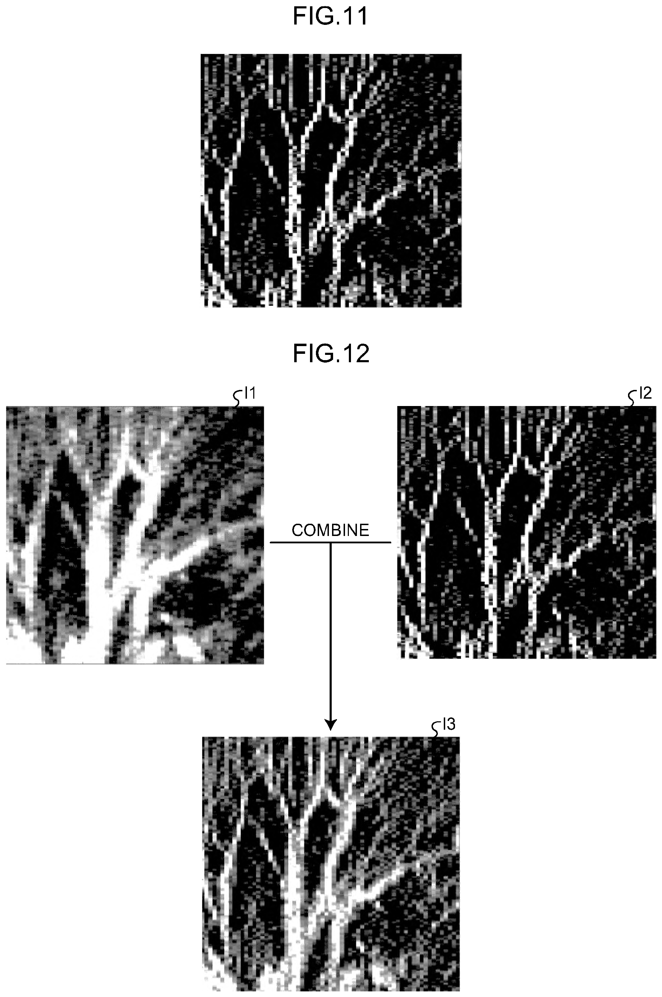

That is, for the respective sets of first ultrasound data generated successively, the execution function 115 successively generates sets of second ultrasound data whose signal intensities have been corrected according to the numbers of frames added. In processing performed when the number of frames collected is still small, if some gain correction is performed, the entire image increases in brightness in a state where gain has been applied excessively, causing flashback to be noticeable. However, signal intensities of the sets of second ultrasound data according to the embodiment are corrected according to the numbers of frames added, so that, for example, as illustrated in FIG. 11, each of the sets of second ultrasound data results in an image in which flashback is adequately minimized.

The first display control function 116 displays, on the display 140, first ultrasound images based on at least sets of first ultrasound data during a period from detection of a mode change until a condition determined beforehand is satisfied (Step S409). For example, the first display control function 116 displays the first ultrasound images on the display 140 during a period from operation for selecting a mode until the condition is satisfied. The first display control function 116 displays, on the display 140, as the first ultrasound image, combined images obtained by combining the first ultrasound data and the second ultrasound data, the combined images having smaller proportions of the second ultrasound data than the second ultrasound data used in generation of the second ultrasound image.

For example, the first display control function 116 successively displays, on the display 140, first ultrasound images obtained by combining first ultrasound data and second ultrasound data at ratios corresponding to elapse of time, during a period from detection of a mode change until a predetermined time period elapses. Or the first display control function 116 successively displays, on the display 140, first ultrasound images obtained by combining first ultrasound data and second ultrasound data at ratios corresponding to the numbers of frames, during a period from detection of a trigger until the number of frames represented by the first ultrasound data reaches a predetermined number.

For example, as illustrated in FIG. 12, the first display control function 116 displays an ultrasound image I3 (first ultrasound image) on the display 140, the ultrasound image I3 being obtained by combining a set of second ultrasound data I2 whose signal intensities have been corrected using filter information, with a set of first ultrasound data I1 at a combining ratio corresponding to a time elapsed or the number of frames.

That is, the first display control function 116 displays a first ultrasound image on the display 140, the first ultrasound image being obtained by combining the set of second ultrasound data I2 with the set of first ultrasound data (blood flow data) generated by the acquisition function 111 at a combining ratio corresponding to a time elapsed or the number of frames. This combining ratio is set such that the shorter the time elapsed (or the smaller the number of frames), the higher the proportion of the set of first ultrasound data, and the longer the time elapsed (or the larger the number of frames), the lower the proportion of the set of first ultrasound data.

In an example of a case where the condition determined beforehand is "the number of frames acquired: 6", the combining ratio when the number of frames used for the set of second ultrasound data is "the number of frames = 2" may be set as "first ultrasound data: second ultrasound data = 0.8:0.2". Furthermore, the combining ratio in a case where "the number of frames = 3" may be set as "first ultrasound data: second ultrasound data = 0.6:0.4"; the combining ratio in a case where "the number of frames = 4" may be set as "first ultrasound data: second ultrasound data = 0.4:0.6"; and the combining ratio in a case where "the number of frames = 5" may be set as "first ultrasound data: second ultrasound data = 0.2:0.8".

For example, in a case where the number of frames of data used in generation of a set of first ultrasound data is "2" for sequentially generated sets of first ultrasound data, the first display control function 116 displays, on the display 140, a first ultrasound image based on data obtained by combining (by additive averaging) the set of first ultrasound data and a corresponding set of second ultrasound data (a set of second ultrasound data generated based on the set of first ultrasound data) at a combining ratio, "0.8:0.2". The first display control function 116 displays first ultrasound images on the display 140 while changing the combining ratio such that as the number of frames of data used for a set of first ultrasound data increases, the proportion of the set of second ultrasound data increases.

In a case where the number of frames (the number of frames used in generation of a set of first ultrasound data in the first embodiment) used in analysis of a signal component of a target (for example, a blood flow signal component) is small, local peak positions may be not extracted in the frames and visibility of the blood flow in the set of second ultrasound data is thus reduced. In the ultrasound diagnostic apparatus 10 according to the embodiment, visibility is improved by combining such second ultrasound data with blood flow data before the local peak position extraction. Furthermore, in the above described example, combining at a combining ratio corresponding to the number of frames added enables output of display data adequately representing results of high-definition processing.

At Step S404, if the condition determined beforehand is satisfied after the detection of the mode change (Step S404, Yes), the processing circuitry 110 executes processing similar to the processing described with respect to Steps S304 to S307 in FIG. 5. That is, the processing circuitry 110 acquires sets of first ultrasound data from the frames of data collected (S410), extracts sets of identification information from the sets of first ultrasound data (Step S411), generates filter information using the sets of identification information (Step S412), and generates sets of second ultrasound data using the filter information generated (Step S413).

The second display control function 117 displays, on the display 140, second ultrasound images based on at least the sets of second ultrasound data after the condition determined beforehand is satisfied (Step S414). Specifically, the second display control function 117 displays, on the display 140, as the second ultrasound images, combined images obtained by combining the sets of first ultrasound data and the sets of second ultrasound data, the combined images having smaller proportions of the sets of first ultrasound data than the sets of first ultrasound data used in generation of the first ultrasound images.

In an example of the case where the condition determined beforehand is "the number of frames acquired: 6" (in the case described with respect to FIG. 6), the second display control function 117 displays a second ultrasound image based on data obtained by combining a set of first ultrasound data and a set of second ultrasound data at a combining ratio, "first ultrasound data: second ultrasound data = 0:1". That is, in a case where the condition determined beforehand is satisfied after the detection of the trigger, the second display control function 117 in the ultrasound diagnostic apparatus 10 causes display of an ultrasound image obtained by high-definition processing as it is. Even after the condition determined beforehand is satisfied, a second ultrasound image based on data obtained by combining a set of first ultrasound data and a set of second ultrasound data at a predetermined combining ratio (for example, first ultrasound data: second ultrasound data = 0.05:0.95) may be displayed.

The above mentioned combining ratios are just examples, and any combining ratios may be set. That is, different combining ratios may be set per frame, but may also be set per any number of frames. Furthermore, with respect to the example, the case where sets of added data (sets of first ultrasound data) are set to be generated in units of six frames has been described above, but the number of frames set is not necessarily six and any number of frames may be set. The combining ratios are respectively set according to the numbers of frames set.

After Step S409 or S414, the processing circuitry 110 determines whether or not to end the display (S415). For example, the processing circuitry 110 determines whether or not operation for ending the second ultrasound data generation mode has been received. In a case where the processing circuitry 110 determines that the display is to be ended (Step S415, Yes), the processing circuitry 110 ends the processing. The processing circuitry 110 continues the processing until the processing circuitry 110 determines that the processing is to be ended (Step S415, No).

As described above, according to the first embodiment, the ultrasound probe 5 transmits ultrasound to a subject and receives ultrasound reflected by the subject. On the basis of reception signals of ultrasound received by the ultrasound probe 5, the acquisition function 111 successively acquires sets of first ultrasound data in the time direction, the sets of first ultrasound data representing a fluid moving in the body of the subject. The execution function 115 executes processing of generating, on the basis of the sets of first ultrasound data successively acquired by the acquisition function 111, sets of second ultrasound data having higher spatial resolution for the fluid than the sets of first ultrasound data. The detection function 112 detects a trigger for executing the processing of generating. The first display control function 116 displays, on a display, first ultrasound images based on at least the sets of first ultrasound data during a period from detection of the trigger until a condition determined beforehand is satisfied. The second display control function 117 displays, on the display, second ultrasound images based on at least the sets of second ultrasound data after the condition determined beforehand is satisfied. Therefore, the ultrasound diagnostic apparatus 10 according to the first embodiment is capable of displaying the first ultrasound images based on the sets of first ultrasound data during the period from the detection of the trigger until the condition is satisfied and enables highly visible images to be provided stably.

Furthermore, according to the first embodiment, the first display control function 116 displays, on the display 140, as the first ultrasound images, combined images obtained by combining the sets of first ultrasound data and the sets of second ultrasound data, the combined images having smaller proportions of the sets of second ultrasound data than the sets of second ultrasound data used in generation of the second ultrasound images. Therefore, the ultrasound diagnostic apparatus 10 according to the first embodiment enables correction of the sets of second ultrasound data with the sets of first ultrasound data.

Furthermore, according to the first embodiment, the second display control function 117 displays, on the display 140, as the second ultrasound images, combined images obtained by combining the sets of first ultrasound data and the sets of second ultrasound data, the combined images having smaller proportions of the sets of first ultrasound data than the sets of first ultrasound data used in generation of the first ultrasound images. Therefore, the ultrasound diagnostic apparatus 10 according to the first embodiment enables highly visible images to be provided stably.

Furthermore, according to the first embodiment, the first display control function 116 successively displays, on the display 140, the first ultrasound images obtained by combining the sets of first ultrasound data and the sets of second ultrasound data at ratios corresponding to elapse of time, during a period from the detection of the trigger until a predetermined time period elapses. Therefore, the ultrasound diagnostic apparatus 10 according to the first embodiment enables display of the first ultrasound images according to the times elapsed from the detection of the trigger.

Furthermore, according to the first embodiment, the first display control function 116 successively displays, on the display 140, the first ultrasound images obtained by combining the sets of first ultrasound data and the sets of second ultrasound data at ratios corresponding to the numbers of frames, during a period from the detection of the trigger until the number of frames represented by the sets of first ultrasound data reaches a predetermined number. Therefore, the ultrasound diagnostic apparatus 10 according to the first embodiment enables display of the first ultrasound images according to the numbers of frames after the detection of the trigger.

Furthermore, according to the first embodiment, the detection function 112 detects, as the trigger, operation for selecting a mode in which the second ultrasound images are to be displayed. The first display control function 116 displays the first ultrasound images on the display 140 during a period from the operation for selecting the mode until the condition is satisfied. Therefore, the ultrasound diagnostic apparatus 10 according to the first embodiment enables observation of an image without discomfort immediately after mode switching.

Furthermore, according to the first embodiment, the generation function 114 generates filter information based on sets of identification information identifying positions of the fluid in the sets of first ultrasound data. The execution function 115 generates the sets of second ultrasound data using the filter information that has been subjected to weighting according to times elapsed since the start of the generation of the sets of second ultrasound data or the numbers of frames of data used in generation of the sets of second ultrasound data. Therefore, the ultrasound diagnostic apparatus 10 according to the first embodiment enables correction related to signal intensities.

Furthermore, according to the first embodiment, the generation function 114 generates filter information that has been weighted such that the shorter the time elapsed or the smaller the number of frames, the smaller the value generated. Therefore, the ultrasound diagnostic apparatus 10 according to the first embodiment enables correction of signal intensities according to the state of collection of the frames of data.

Furthermore, according to the first embodiment, the acquisition function 111 acquires, as the sets of first ultrasound data, sets of added data each obtained by addition processing of a plurality of frames of data that are obtained by transmission and reception of the ultrasound by the ultrasound probe 5 and that are consecutive in the time direction. Therefore, the ultrasound diagnostic apparatus 10 according to the first embodiment enables extraction of a signal component representing the fluid (for example, blood flow) in the subject from ultrasound data having a high SN ratio.

### First Modified Example

The case where high-definition processing is started triggered by a mode transition has been described above with respect to the first embodiment, but the embodiment is not limited to this case, and high-definition processing may be started on the basis of other information.

For example, the ultrasound diagnostic apparatus 10 may perform control such that, after transitioning to a mode in which high-definition processing is performed at Step S102 in FIG. 2, high-definition processing from Step S403 onward is started using, as a trigger, a state where a change in the position of a region in successively collected ultrasound data (frames of data) no longer occurs. That is, processing related to high-definition may be started using, as a trigger, stoppage of body motion of the subject and stoppage of motion of the ultrasound probe 5.

In this case, the detection function 112 detects, as the trigger, detection of a stationary state of the ultrasound probe 5. Furthermore, the detection function 112 detects, as the trigger, a stopped state of the body motion represented by ultrasound data of the subject acquired through preprocessing of sets of first ultrasound data. For example, by detecting a change in the position of a region included in the ultrasound data, the detection function 112 determines the stationary state of the ultrasound probe 5 and the stopped state of the body motion. For example, the detection function 112 compares the amount of displacement of tissue between frames calculated at Step S402 in FIG. 8 with a threshold, and determines that the position of the region has changed in a case where the amount of displacement is equal to larger than the threshold. On the contrary, in a case where the amount of displacement is smaller than the threshold, the detection function 112 determines that the position of the region has not changed.

The processing circuitry 110 starts processing of Step S403 on a condition that the detection function 112 determines that the position of the region has not changed. The processing circuitry 110 thereby starts high-definition processing after the change in the position of the region falls below the threshold (after the ultrasound probe 5 enters the stationary state and the body motion enters the stopped state). That is, the first display control function 116 displays first ultrasound images on the display 140 during a period from the detection of the stationary state of the ultrasound probe 5 until a condition is satisfied. Furthermore, the first display control function 116 displays the first ultrasound images on the display 140 during a period from the detection of the stopped state of the body motion until the condition is satisfied.

As described above, the ultrasound diagnostic apparatus 10 according to the first modified example is capable of starting collecting ultrasound data triggered by a mode transition and starting high-definition processing triggered by a state where a change in the position of a region in the ultrasound data no longer occurs. A method of detecting the motion of the region is not limited to the processing mentioned above and optical flow or template matching may be used instead.

As described above, according to the first modified example, the detection function 112 detects, as a trigger, detection of the stationary state of the ultrasound probe 5. Furthermore, the detection function 112 detects, as the trigger, the stopped state of the body motion represented by ultrasound data of the subject acquired through preprocessing of sets of first ultrasound data. The first display control function 116 displays first ultrasound images on the display 140 during a period from detection of the stationary state of the ultrasound probe 5 until a condition is satisfied. Furthermore, the first display control function 116 displays first ultrasound images on the display 140 during a period from detection of the stopped state of the body motion until the condition is satisfied. Therefore, the ultrasound diagnostic apparatus 10 according to the first modified example is capable of performing high-definition processing using ultrasound data (frames of data) suitable for high-definition processing, and enables highly visible images to be provided stably.

### Second Modified Example

With respect to the first embodiment, the case where the filter information and the combining ratio are dynamically changed until the number of frames of data used for a set of first ultrasound data (added data) reaches a set number of frames has been described above. However, the embodiment is not limited to this case, and the filter information and the combining ratio may be dynamically changed even after frames of data for a sufficient number of frames have been collected.

For example, in displaying a second ultrasound image at Step S414 of FIG. 8, after the second ultrasound image (for example, the second ultrasound image after the number of frames added reaches six) is displayed using a set number of frames, the ultrasound diagnostic apparatus 10 may perform control to evaluate image quality of the second ultrasound image and to change the filter information and the combining ratio again in accordance with a result of this evaluation.

In this case, the detection function 112 detects the image quality of the second ultrasound image. For example, as described with respect to the first modified example, the detection function 112 detects a change in the position of a target by comparing the amount of displacement of tissue between frames calculated at Step S402 of FIG. 8 with a threshold. In response to detection of a change in the position of the target, high-definition processing is executed using frames of data collected in a state after the change. That is, the processing described with respect to the first embodiment is executed again, and the first display control function 116 successively displays, on the display 140, first ultrasound images obtained by combining sets of first ultrasound data and sets of second ultrasound data at ratios corresponding to image quality index values, during a period from detection of a trigger until the image quality index value represented by the set of second ultrasound data enters a predetermined state. The image quality of the second ultrasound images is not limited to the above mentioned change in the position and another known image quality index may be used instead.

As described above, according to the second modified example, the first display control function 116 successively displays, on the display 140, first ultrasound images obtained by combining sets of first ultrasound data and sets of second ultrasound data at ratios each corresponding to an image quality index value, during a period from detection of a trigger until the image quality index value represented by the set of second ultrasound data enters a predetermined state. Therefore, the ultrasound diagnostic apparatus 10 according to the second modified example is capable of executing processing according to image quality by evaluating image quality of a second ultrasound image during execution of high-definition processing, and enables display of ultrasound images that are even more highly visible.

### Second Embodiment

The case where added data obtained by execution of addition processing of a plurality of frames of data are used as a set of first ultrasound data has been described above with respect to the first embodiment. In a case described with respect to a second embodiment, frames of data collected are used as sets of first ultrasound data. That is, a case where signals representing a target (a fluid moving in a subject) are extracted from each of a plurality of frames of data and combining processing is performed on signal components extracted will be described with respect to the second embodiment. In the second embodiment, processing executed by the acquisition function 111, processing executed by the extraction function 113, processing executed by the first display control function 116, and processing executed by the second display control function 117 are different from those of the first embodiment. These will be described mainly hereinafter.

The acquisition function 111 according to the second embodiment acquires, as a set of first ultrasound data, each of a plurality of frames of data successively collected on the basis of transmission and reception of ultrasound by the ultrasound probe 5. For example, the acquisition function 111 acquires each frame of data after the processing of Step S403 in FIG. 8 as a set of first ultrasound data.

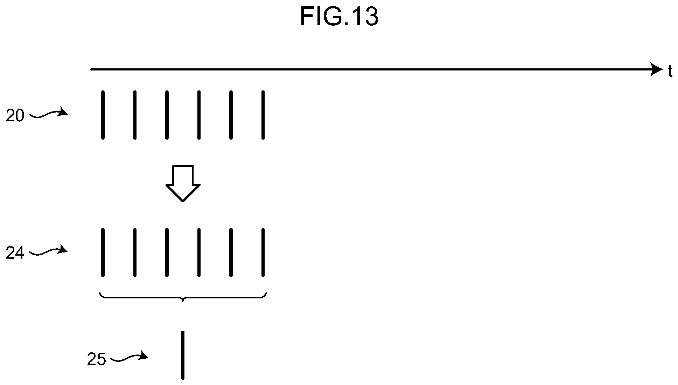

The extraction function 113 according to the second embodiment respectively extracts signal components (local peak signals) representing local peaks of a fluid in individual frames, from the plurality of frames of data. The signal components representing the local peaks of the fluid are signals each having a relatively high signal value (amplitude) in a local range of the frame. The extraction of the local peak signals may be performed using the kernel 30 used in the first embodiment or by another technique. The extraction function 113 respectively executes extraction processing of local peak signals for the frames of data successively acquired as sets of first ultrasound data. For example, as illustrated in FIG. 13, the extraction function 113 respectively extracts local peak signal components 24 corresponding to six frames by respectively executing extraction processing for the six frames.

The acquisition function 111 generates added data 25 obtained by adding the plurality of local peak signal components 24 respectively extracted from the frames of data by the extraction function 113. Specifically, the acquisition function 111 generates added data obtained by adding the local peak signal components 24 for the frames corresponding to the time elapsed or the number of frames. For example, as illustrated in FIG. 13, the acquisition function 111 adds the six signal components 24 respectively extracted from the six frames and thereby generates the added data 25.

The first display control function 116 according to the second embodiment displays first ultrasound images on the display 140 by combining the plurality of frames of data 20 and the added data 25 generated by the acquisition function 111 at a first combining ratio during a period from detection of a trigger (for example, a mode transition, a state where body motion is stationary, or a state where the ultrasound probe 5 is stationary) until a condition determined beforehand (for example, elapse of a predetermined time period or a predetermined number of frames) is satisfied. For example, the first display control function 116 generates the added data 25 obtained by adding the local peak signal components 24 corresponding to the collected number of frames and combines the added data 25 and the frames of data 20 at the first combining ratio until the six frames illustrated in FIG. 13 are collected. The first display control function 116 displays, on the display 140, the first ultrasound images obtained by combining the added data 25 and the frames of data 20 at the first combining ratio. In the first combining ratio, the frames of data 20 have a higher proportion than the added data 25.

The first combining ratio may be changed according to the time elapsed or number of frames. For example, the combining ratio may be changed such that as time elapses (or as the number of frames increases), the proportion of the added data 25 increases and the proportion of the frames of data 20 decreases. Specifically, in a case where two frames have been collected, combined data obtained by combining added data 25 and frames of data 20 at a first combining ratio, "2:8", may be generated, and in a case where three frames have been collected, combined data obtained by combining added data 25 and frames of data 20 at a first combining ratio, "4:6", may be generated. The first display control function 116 may display, on the display 140, first ultrasound images based on only frames of data 20, without using added data 25, during a period from detection of a trigger until a condition determined beforehand is satisfied.

On the other hand, after a trigger (for example, a mode transition, a state where body motion is stationary, or a state where the ultrasound probe 5 is stationary) is detected and a condition determined beforehand (for example, elapse of a predetermined period or a predetermined number of frames) is satisfied, the second display control function 117 according to the second embodiment combines a plurality of frames of data 20 and added data 25 generated by the acquisition function 111 at a second combining ratio and thereby displays a second ultrasound image on the display 140. For example, the second display control function 117 generates the added data 25 obtained by adding the local peak signal components 24 corresponding to the collected number of frames and combines the added data 25 and the frames of data 20 at the second combining ratio after the six frames illustrated in FIG. 13 have been collected. The second display control function 117 displays, on the display 140, the second ultrasound image acquired by combining the added data 25 and the frames of data 20 at the second combining ratio. In the second combining ratio, the frames of data 20 have a lower proportion than the added data 25. The second display control function 117 may display, on the display 140, a second ultrasound image based on the added data 25, without using the plurality of frames of data 20.

As described above, according to the second embodiment, even in a case where high-definition processing of extracting local peak signals from each of a plurality of frames of data 20 is performed, during a period from detection of a trigger (for example, a mode transition, a state where body motion is stationary, or a state where the ultrasound probe 5 is stationary) until a condition determined beforehand (for example, elapse of a predetermined time period or a predetermined number of frames) is satisfied, first ultrasound images based on at least the plurality of frames of data 20 are displayed on the display 140, and after the condition determined beforehand is satisfied, second ultrasound images based on at least added data 25 is displayed on the display 140. Highly visible images are thereby able to be provided stably.

Local peak signals may be extracted respectively as signal components (local peak signals) representing local peaks of a fluid in individual frames by performing processing of sharpening local peaks on a plurality of frames of data 20 (sets of first ultrasound data). For example, the extraction function 113 performs processing of resampling each of a plurality of frames of data 20 (sets of first ultrasound data) to increase pixel density and increase effective resolution. For example, this resampling may replace each pixel with a plurality of pixels smaller in size. The signal value of each pixel after the replacement may be interpolated from an original signal value (a signal value before the replacement) by, for example, bicubic interpolation. Furthermore, the extraction function 113 sharpens local peak signals by raising pixel values represented by individual frames of data 20, after their effective resolution is increased, to a power (for example, the 8th power or the 12th power). That is, by raising pixel values to a power, a pixel having a pixel value higher than surrounding pixel values is extracted as a pixel representing a local peak of a fluid. The extraction function 113 extracts local peak signal components 24 corresponding to the individual frames by performing threshold processing on the plurality of frames of data 20 whose pixel values have been sharpened. The acquisition function 111 generates added data 25 obtained by adding the plurality of local peak signal components 24 respectively extracted from the frames of data by the extraction function 113.

In this case also, the first display control function 116 display, on the display 140, first ultrasound images based on at least a plurality of frames of data 20 during a period from detection of a trigger (for example, a mode transition, a state where body motion is stationary, or a state where the ultrasound probe 5 is stationary) until a condition determined beforehand (elapse of a predetermined time period or a predetermined number of frames) is satisfied. On the other hand, after the condition determined beforehand is satisfied, the second display control function 117 displays, on the display 140, second ultrasound images based on at least added data 25. Highly visible images are thereby able to be provided stably.

The examples in which the above described embodiments are applied to ultrasound diagnostic apparatuses have been described above, but the embodiments are not limited to these example, and may be applied to medical information processing apparatuses other than ultrasound diagnostic apparatuses. For example, the embodiments are applicable to a medical information processing apparatus, such as a workstation or a server, which acquires ultrasound data obtained on the basis of results of ultrasound scans of subjects. For example, the embodiments may be applied to a case where a medical information processing apparatus, such as a workstation or a server, executes the above described processing using a plurality of frames of data successively collected.

Furthermore, the example in which the fluid moving in the body of the subject is blood flow (blood) has been described above with respect to the embodiments, but the fluid may be a contrast agent.

The components of the apparatuses have been functionally and conceptually illustrated in the drawings for description of the embodiments and are not necessarily configured physically as illustrated in the drawings. That is, specific forms of separation and integration of the apparatuses are not limited to those illustrated in the drawings, and all or part thereof may be configured to be functionally or physically separated or integrated in any units according to various loads and use situations. In addition, all or any part of the processing functions executed in the apparatuses may be implemented by a CPU and a program analyzed and executed by the CPU, or implemented as hardware by wired logic.

Furthermore, the methods described above with respect to the embodiments may be implemented by a computer, such as a personal computer or a workstation, executing a program that has been prepared beforehand. This program may be provided via a network, such as the Internet. The program may also be recorded on a non-transitory computer-readable recording medium, such as a hard disk, a flexible disk (FD), a CD-ROM, an MO, a DVD, or a flash memory, such as a USB memory or an SD card memory, and executed by being read from the non-transitory recording medium by a computer.

As described above, according to an embodiment, highly visible images are able to be provided stably.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the accompanying claims. The accompanying claims are intended to cover such forms or modifications as would fall within the scope of the inventions.

## Claims

1. An ultrasound diagnostic apparatus (10), comprising:
an ultrasound probe (5) configured to transmit ultrasound to a subject and receive ultrasound reflected by the subject;
an acquisition unit (111) configured to successively acquire, on the basis of reception signals of the ultrasound received by the ultrasound probe (5), first ultrasound data in a time direction, the first ultrasound data representing a fluid moving in the body of the subject;
an execution unit (115) configured to execute processing of generating, on the basis of the first ultrasound data successively acquired by the acquisition unit (111), second ultrasound data having higher spatial resolution for the fluid than the first ultrasound data;
a detection unit (112) configured to detect a trigger for executing the processing;
a first display control unit (116) configured to display, on a display, first ultrasound image based on at least the first ultrasound data, during a period from detection of the trigger until a condition determined beforehand is satisfied; and
a second display control unit (117) configured to display, on the display, second ultrasound image based on at least the sets of second ultrasound data, after the condition determined beforehand is satisfied.

2. The ultrasound diagnostic apparatus (10) according to claim 1, wherein the first display control unit (116) is configured to display, on the display, as the first ultrasound image, combined image obtained by combining the first ultrasound data and the second ultrasound data, the combined image having smaller proportions of the second ultrasound data than the second ultrasound data used in generation of the second ultrasound image.

3. The ultrasound diagnostic apparatus (10) according to claim 2, wherein the second display control unit (117) is configured to display, on the display, as the second ultrasound image, combined image obtained by combining the first ultrasound data and the second ultrasound data, the combined image having smaller proportions of the first ultrasound data than the first ultrasound data used in generation of the first ultrasound image.

4. The ultrasound diagnostic apparatus (10) according to claim 2, wherein the first display control unit (116) is configured to successively display, on the display, the first ultrasound images obtained by combining the first ultrasound data and the second ultrasound data at ratios corresponding to elapse of time, during a period from the detection of the trigger until a predetermined time period elapses.

5. The ultrasound diagnostic apparatus (10) according to claim 2, wherein the first display control unit (116) is configured to successively display, on the display, the first ultrasound images obtained by combining the first ultrasound data and the second ultrasound data at ratios corresponding to the numbers of frames, during a period from the detection of the trigger until the number of frames represented by the first ultrasound data reaches a predetermined number.

6. The ultrasound diagnostic apparatus (10) according to claim 2, wherein the first display control unit (116) is configured to successively display, on the display, the first ultrasound images obtained by combining the first ultrasound data and the second ultrasound data at ratios each corresponding to an image quality index value, during a period from the detection of the trigger until the image quality index value represented by the second ultrasound data enters a predetermined state.

7. The ultrasound diagnostic apparatus (10) according to claim 1, wherein
the detection unit (112) is configured to detect, as the trigger, operation for selecting a mode in which the second ultrasound image are displayed, and
the first display control unit (116) is configured to display the first ultrasound image on the display during a period from the operation for selecting the mode until the condition is satisfied.

8. The ultrasound diagnostic apparatus (10) according to claim 7, wherein
the detection unit (112) is configured to detect, as the trigger, detection of a stationary state of the ultrasound probe (5), and
the first display control unit (116) is configured to display the first ultrasound image on the display during a period from the detection of the stationary state of the ultrasound probe (5) until the condition is satisfied.

9. The ultrasound diagnostic apparatus (10) according to claim 1, wherein
the detection unit (112) is configured to detect, as the trigger, a stopped state of body motion represented by ultrasound data of the subject acquired through preprocessing of the first ultrasound data, and
the first display control unit (116) is configured to display the first ultrasound image on the display during a period from detection of the stopped state of the body motion until the condition is satisfied.

10. The ultrasound diagnostic apparatus (10) according to claim 1, further comprising:
a generation unit (114) configured to generate filter information based on identification information identifying positions of the fluid in the first ultrasound data, wherein
the execution unit (115) is configured to generate the second ultrasound data using the filter information that has been subjected to weighting according to times elapsed since start of generation of the second ultrasound data or the numbers of frames of data used in generation of the second ultrasound data.

11. The ultrasound diagnostic apparatus (10) according to claim 10, wherein the generation unit (114) is configured to generate the filter information that has been weighted such that the shorter the time elapsed or the smaller the number of frames of data, the smaller the value generated.

12. The ultrasound diagnostic apparatus (10) according to any one of claims 1 to 11, wherein the acquisition unit (111) is configured to acquire, as the first ultrasound data, added data each obtained by addition processing of a plurality of frames of data that are obtained by transmission and reception of the ultrasound by the ultrasound probe (5) and that are consecutive in the time direction.

13. A storage medium that stores a program for causing a computer to execute processing including:
successively acquiring, in a time direction, first ultrasound data representing a fluid moving in the body of a subject;
executing processing of acquiring, on the basis of the first ultrasound data successively acquired, second ultrasound data having higher spatial resolution for the fluid than the first ultrasound data;
detecting a trigger for executing the processing;
displaying, on a display, first ultrasound image based on at least the first ultrasound data, during a period from detection of the trigger until a condition determined beforehand is satisfied; and
displaying, on the display, second ultrasound image based on at least the second ultrasound data, after the condition determined beforehand is satisfied.
